# EUROPÄISCHE PATENTSCHRIFT

(11) **EP 1 640 042 B1**
(45) Veröffentlichungstag und Bekanntmachung des Hinweises auf die Patenterteilung: **30.12.2009**
(21) Anmeldenummer: 05090245.1
(22) Anmeldetag: 24.08.2005
(51) Int. Cl.: A61Q 19/02, A61K 8/04, A61K 8/73, A61K 8/66, A61K 8/97

(54) **Kosmetisches Aufhellungs- und Reinigungsmittel für die Haut enthaltend Extrakte aus Lotus, Kiwi, Orchideen und Süssholz**
Cosmetic skin-whitening and cleansing composition comprising extracts from lotus, kiwi, orchids and liquorice
Composition cosmétique pour le blanchissement et le nettoyage de la peau comprenant des extraits de lotus, de kiwi, d'orchidées et de reglisse

(30) Priorität: 27.08.2004 DE 102004042299
(43) Veröffentlichungstag der Anmeldung: 29.03.2006
(73) Patentinhaber: COTY PRESTIGE LANCASTER GROUP GmbH, 55116 Mainz (DE)
(72) Erfinder: Golz-Berner, Karin, 98000 Monaco (MC); Zastrow, Leonhard, 98000 Monaco (MC)
(74) Vertreter: Walter, Wolf-Jürgen

(56) Entgegenhaltungen:
- WO-A-2004/093839
- WO-A-2005/041915
- DE-A1- 4 227 806
- DE-A1- 19 715 478
- DE-C1- 10 139 612
- US-B1- 6 468 564
- PATENT ABSTRACTS OF JAPAN Bd. 017, Nr. 025 (C-1017), 18. Januar 1993 (1993-01-18) & JP 04 247012 A (MIKIMOTO SEIYAKU KK), 3. September 1992 (1992-09-03)
- SALEEM A ET AL: "TOTAL PHENOLICS CONCENTRATION AND ANTIOXIDANT POTENTIAL OF EXTRACTS OF MEDICINAL PLANTS OF PAKISTAN" ZEITSCHRIFT FUER NATURFORSCHUNG. C, A JOURNAL OF BIOSCIENCES, TUEBINGEN, DE, Bd. 56, Nr. 11/12, 2001, Seiten 973-978, XP009045385 ISSN: 0939-5075
- NN: "GATTEFOSSE - WITH IN VITRO SUBSTANTIATED CLAIMS URL - http://www.gattefosse.com/cosmetic/mainvit ext.htm DWWW- 2004-08-11" GATTEFOSSE, 11. August 2004 (2004-08-11), XP002292066
- PATENT ABSTRACTS OF JAPAN Bd. 2002, Nr. 11, 6. November 2002 (2002-11-06) & JP 2002 205933 A (ICHIMARU PHARCOS CO LTD), 23. Juli 2002 (2002-07-23)
- FU GUO-QIANG ET AL: "Inhibitory effects of ethanolic extracts from 196 kind of Chinese herbs on tyrosinase" BIOSIS, 2003, XP002292067
- DATABASE WPI Section Ch, Week 200225 Derwent Publications Ltd., London, GB; Class B04, AN 2002-189009 XP002360136 "Preparation of beautifying health cosmetics containing special jewel, jade and medicinal stone fine powder" & CN 1 324 609 A (NIU H) 5. Dezember 2001 (2001-12-05)
- DATABASE WPI Section Ch, Week 200301 Derwent Publications Ltd., London, GB; Class B04, AN 2003-000282 XP002283254 "New 'Baikuangkangti' powder used for e.g. reducing blood pressure and blood fat and losing weight, comprises a mixture of different minera including jade dust, magnetite, multi-metal ore, rare-earth ore, edible salt and mica" & CN 1 363 282 A (YU B) 14. August 2002 (2002-08-14)

## Beschreibung

Die Erfindung betrifft ein kosmetisches Aufhellungs- und Reinigungsmittel für die Haut, dessen Basis ein Gemisch von Pflanzenextrakten ist.

Die hautaufhellende Wirkung von Pflanzenextrakten ist bekannt. In der WO 98/58628 werden auch verschiedene pflanzliche Aufhellungsmittel allein oder in Kombination miteinander beschrieben, wie Maulbeerenextrakt, Bärentraubenextrakt oder Süßholzextrakt.

Die Wirkung derartiger Extrakte sowohl allein als auch in Kombination miteinander oder mit Kojisäure oder Arbutin ist nicht immer befriedigend, da sie oftmals nur sehr kurzzeitig auftritt.

Die JP 04-247012 (Abstract) offenbart den Einsatz von Blattextrakten von Lotus für Präparate mit verschiedenen Wirkungen.

Die US 6468564 beschreibt eine topische Zusammensetzung, die einen Extrakt der Samen von Lotus enthält und Antialterungs- und Antifaltenwirkung erzielen soll.

Die DE 10139612 offenbart ein hautglättendes Kosmetikum, das neben 5 weiteren Bestandteilen auch Lotusblütenextrakt enthält.

In Z. Naturforsch. 56c, 973-978 (2001) werden 7 unterschiedliche Pflanzenextrakte als Antioxidantien aufgeführt, darunter auch Nymphea lotus.

Das Produktblatt Gatefosse XP002292066 gibt an, welche Standard extrakte angeboten werden, darunter auch Kiwi-Extrakt ohne nähere Herkunftsangabe.

Der Abstract von JP 2002-205933 lässt erkennen, dass Pflanzenextrakte aus der Orchideenfamilie als Antioxidant, Feuchthaltemittel und kosmetische Zusammensetzung verwendet werden, u.a. mit verbessernder Wirkung auf Sommersprossen.

In Zhonghua Pifuke Zazhi, Feb. 2003, XP 002292067 beschreiben die Autoren, dass 20 Arten von Rohextrakten aus chinesischen Pflanzen als Depigmentierungsmittel für Hautkrankheiten durch anormale Tyrosinaseaktivität verwendet werden können, darunter auch Gastrodia elata, eine Orchideenart.

Die DE 4227806 beschreibt ein topisches Hautaufhellungsmittel aus einem Flavonoid (z.B. Ascorbinsäure) und einem DOPA-Chinon-Reduktionsmittel wie Süßholzextrakt mit synergistischer Wirkung.

Der Erfindung liegt die Aufgabe zugrunde, ein kosmetisches Aufhellungsmittel bereitzustellen, das zugleich eine gewisse Reinigungswirkung zeigt und dessen Aufhellungswirkung langanhaltend ist.

Erfindungsgemäß umfasst das neue kosmetisches Aufhellungs- und Reinigungsmittel für die Haut einen Komplex aus Pflanzenextrakten, bestehend aus 38-62 Gew-% Lotusblütenextrakt, 22-42 Gew-% Kiwi-Fruchtextrakt, 15-26 Gew-% Orchideenextrakt der weißen Orchidee Phalaenopsis amabilis und 0,001-2 Gew-% Süßholzextrakt, bezogen auf das Trockengewicht des Komplexes. Der Komplex nimmt einem Anteil von 0,5 bis 5 Gew-% des Aufhellungsmittels ein, und der Rest bis zu 100 Gew-% des Mittels sind übliche kosmetische Trägerstoffe, Hilfsstoffe, andere Wirkstoffe oder Gemische davon.

Der Extrakt der Lotuspflanze, insbesondere von Lotusblüten, der mittels eines mehrwertigen Alkohols, wie z.B. Propylenglycol, gewonnen wird oder als Alkohol/Wasser-Gemisch, erhalten bei ca. 15 bis 40 °C, zeigt für sich antiradikalische und die Blutkapillaren stärkende Eigenschaften und verbessert die Mikrozirkulation in der Haut. Weiterhin wird die mikrobielle Vermehrung reduziert und damit die Sebumproduktion auf der Hautoberfläche begrenzt.

Die Lotusblume (Nelumbo nucifera) ist aus der traditionellen chinesischen Medizin als Heilpflanze bekannt, wobei Blätter, Blattstiele, Rhizome, Staubblätter und Samen bei bestimmten Krankheitserscheinungen, wie Blutungen, Bluthochdruck, Schlaflosigkeit sowie als Herztonikum eingesetzt werden.

Zusammen mit einem Extrakt einer weißen Orchidee, insbesondere der Orchidee Phalaenopsis amabilis, einem Süßholzextrakt (Glycyrrhiza glabra) und einem Kiwi-Extrakt (Actinidia chinensis) wird mit dem Lotusblumenextrakt in der Gesamtkombination eine besonders langanhaltende Aufhellungswirkung für die Hautoberfläche bei gleichzeitiger Reinigungswirkung erreicht. Diese Wirksamkeit wurde durch Consumer-Tests bestätigt. Dabei werden insbesondere dunklere fleckenartige Hautstellen gegenüber helleren aufgehellt, so dass sich insgesamt ein gleichmäßigere Aufhellung ergibt.

In Bezug auf den Orchideen-Extrakt ist bekannt, dass allein die Art Phalaenopsis etwa 40 Spezies in mehr als hundert verschiedenen Farben umfaßt. Diese Art ist im tropischen Asien bis Südindien beheimatet. Die weiße Orchidee Phalaenopsis amabilis wird in Burma, Buthan und im südlichen Indien gefunden.

Es wird ein Extrakt der oberirdischen Teile dieser Pflanze, d.h. der Blätter, Blüten und Stengel eingesetzt. Dieser Extrakt kann ein wäßriger oder alkoholischer oder wäßrig/alkoholischer Extrakt sein. Als Alkohole können niedere einwertige Alkohole wie Ethanol, Propanol, Isopropanol oder Gemische davon eingesetzt werden, oder es werden mehrwertige Alkohole wie z.B Propylenglycol eingesetzt. Die Extraktionstemperatur liegt im Bereich von 10 bis 40 °C. Der erhaltene Extrakt kann in flüssiger oder - nach Sprüh- oder Gefriertrocknung beispielsweise - auch in fester Form in den Komplex eingebracht werden. Bei Einbringen in fester Form sind Puderteilchen mit Teilchengrößen im Bereich von 0,1 bis 100 µm bevorzugt.

Der erfindungsgemäß eingesetzte Süßholzextrakt ist ein handelsüblicher wässrig-alkoholischer Wurzelextrakt.

Der erfindungsgemäß eingesetzte Kiwi-Extrakt ist ebenfalls ein handelsüblicher wässrig-alkoholischer Fruchtextrakt oder liegt in Pulverform vor.

Die Extrakte können sowohl in verkapselter Form als auch unverkapselt in die Formulierung eingebracht werden. Als Verkapselungsmaterial sind verschieden Arten von Liposomen oder andere bekannte Mikrokapseln wie solche aus Lecithin (Phospholipide), Carboxymethycellulose und anderen Materialien einsetzbar.

Bevorzugt ist, dass der Komplex 46-55 Gew-% Lotusextrakt enthält.

Ebenfalls bevorzugt ist, dass der Komplex 27-37 Gew-% Kiwi-Fruchtextrakt enthält.

Weiterhin bevorzugt ist, dass der Komplex 17-21 Gew-% Orchideenextrakt enthält.

Weiterhin bevorzugt ist, dass der Komplex 0,005-0,9 Gew-% Süßholzextrakt enthält, insbesondere 0,01-0,5 Gew-%.

In einer weiteren bevorzugten Ausführungsform enthält der Komplex als Lotusextrakt einen Lotusblütenextrakt. Die damit erreichte Aufhellung ist überraschend noch etwas länger anhaltend als die mit dem Lotusextrakt erreichte.

Das Kosmetikum kann weitere Wirkstoffe enthalten. Zu den bevorzugten kosmetischen Wirkstoffen gehören z. B. anorganische und organische Lichtschutzmittel, Radikalfänger wie z.B. einen RPF-Komplex mit hohem Radikalschutzfaktor gemäß WO99/66881, sowie Feuchthaltemittel, Erweichungsmittel, Vitamine, Enzyme, andere pflanzliche Wirkstoffe, Polymere, Antioxidationsmittel, entzündungswidrige natürliche Wirkstoffe, mit Sauerstoff beladene asymmetrische lamellare Aggregate gemäß WO 94/00109; Aufschlußprodukte von Hefen oder pflanzlichen Stoffen, hergestellt durch ein schonendes Ultraschall-Aufschlußverfahren gemäß WO 94/13783, Kaolin sowie mit SiO₂ modifiziertes Kaolin gemäß WO94/17588.

Einen weiteren Wirkstoff, den das erfindungsgemäße Kosmetikum enthalten kann, sind fein verteilte hartmagnetische Einbereichsteilchen (Einkristalle) mit hoher Koerzitivfeldstärke von 3000 bis 5000 Oerstedt und mit Korngrößen im Bereich von 50 bis 1200 nm, vorzugsweise 50-250 nm, mit oder ohne die o.g. asymmetrischen lamellaren Aggregate, wobei diese hartmagnetischen Teilchen insbesondere Barium- und/oder Strontiumhexaferrite sind, erzeugt nach der Glaskristallisationstechnik durch Züchtung von Einkristallen aus einer abgeschreckten Glasschmelze (siehe WO95/03061 z.B. Beispiel 2 oder 3; und WO98/44895 z.B. Beispiel 1C). Vorzugsweise werden diese Teilchen zusammen mit Jadepulver eingesetzt, wodurch zugleich zur Aufhellungswirkung die Mikrozirkulationswirkung der bekannten hartmagnetischen Teilchen in kosmetischen Zusammensetzungen deutlich übertroffen und damit der Antransport von Sauerstoff und Nährstoffen sowie der Abtransport von Stoffwechsel-Endprodukten im Hautgewebe verbessert wird. Dies unterstützt die Reinigungswirkung des erfindungsgemäßen Mittels.

Bevorzugt ist dabei eine Kombination von 0,0001-2 Gew-% hartmagnetische Teilchen aus Bariumhexaferrit-Einkristallen mit einer Teilchengröße im Bereich von 100 bis 350 nm und einer Koerzitivkraft der Teilchen im Bereich von 300.000 bis 1.200.000 A/m und und 0,0001 bis 0,7 Gew-% gemahlenem Jadestein mit einer Teilchengröße im Bereich von 50 - 95 nm.

Wenn das neue kosmetische Mittel ein Gemisch von UVA-Sonnenschutzfilter und UVB-Sonnenschutzfilter enthält, dann liegen diese insbesondere im Verhältnis 1:0,6-1,4 vor.

Zu Antioxidationsmitteln gehören Vitamine wie Vitamin C und Derivate davon, beispielsweise Ascorbylacetat, -phosphat und -palmitat; Vitamin A und Derivate davon; Folsäure und deren Derivate, Vitamin E und deren Derivate, wie Tocopherylacetat; Flavone oder Flavonoide; Aminosäuren, wie Histidin, Glycin, Tyrosin, Tryptophan und Derivate davon; Harnsäure und Derivate davon.

Als Erweichungsmittel können normalerweise eine Vielzahl von Verbindungen eingesetzt werden, wie Stearylalkohol, Glycerylmonoricinoleat, Glycerylmonostearat, Propan-1,2-diol, Butan-1,3-diol, Cetylalkohol, Isohexadecan, Isopropylisostearat, Stearinsäure, Isobutylpalmitat, Oleylalkohol, Isopropyllaurat, Decyloleat, Octadecan-2-ol, Isocetylalkohol, Cetylpalmitat, Siliconöle wie Dimethylpolysiloxan, Isopropylmyristat, Isopropylpalmitat, Polyethylenglycol, Lanolin, Kakaobutter, pflanzliche Öle wie Maisöl, Baumwollsamenöl, Olivenöl, mineralische Öle, Butylmyristat, Palmitinsäure usw.

Es ist weiterhin vorteilhaft, den erfindungsgemäßen Zusammensetzungen entsprechende wasser- und/oder öllösliche UVA- oder UVB-Filter oder beide zuzusetzen. Zu vorteilhaften öllöslichen UVB-Filtern gehören 4-Aminobenzoesäure-Derivate wie der 4-(Dimethylamino)-benzoesäure-(2-ethylhexyl)ester; Ester der Zimtsäure wie der 4-Methoxyzimtsäure(2-ethylhexyl)ester, Benzophenon-Derivate wie 2-Hydroxy-4-methoxybenzophenon; 3-Benzylidencampher-Derivate wie 3-Benzylidencampher.

Wasserlösliche UVB-Filter sind z.B. Sulfonsäurederivate von Benzophenon oder von 3-Benzylidencampher oder Salze wie das Na- oder K-Salz der 2-Phenylbenzimidazol-5-sulfonsäure.

Zu UVA-Filtern gehören Dibenzoylmethan-Derivate wie 1-Phenyl-4-(4'-isopropylphenyl)propan-1,3-dion, Butyl Methoxybenzöyl-methane oder Menthyl Anthranilate.

Besonders bevorzugt sind Benzophenone-3, Butyl Methoxydibenzoylmethane, Octyl Methoxycinnamate, Octyl Salicylate, 4-Methylbenzylidene Camphor, Homosalate, Octocrylene, Ethylhexyl Methoxycinnamate, Isoamyl-p-Methoxycinnamate, Octyl Dimethyl PABA, Ethylhexyltriazone, Diethylhexyl Butamido Triazone, Ethylhexyl Salicylate, Methylene Bos-Benzotriazolyl Tetramethylbutylphenol, Disodium Phenyl Dibenzimidazole Tetrasulfonate, Bis-Ethylhexyloxyphenol Methoxyphenyl Triazine.

Weiterhin einsetzbar als Sonnenschutzfilter sind anorganische Pigmente auf Basis von Metalloxiden, wie TiO₂, SiO₂, ZnO, Fe₂O₃, ZrO₂, MnO, Al₂O₃, die auch im Gemisch verwendet werden können.

Bevorzugt enthält das erfindungsgemäße Mittel ein Gemisch von UVA-Sonnenschutzfilter und UVB-Sonnenschutzfilter im Verhältnis 1:0,6-1,4, um einen ausreichenden Schutz des Anwenders gegen UV-Strahlung zu gewährleisten.

Besonders bevorzugt als anorganische Pigmente sind agglomerierte Substrate von TiO₂ und/oder ZnO, die einen Gehalt an sphärischen und porösen SiO₂-Teilchen aufweisen, wobei die SiO₂-Teilchen eine Teilchengröße im Bereich von 0,05 µm bis 1,5 µm haben, und neben den SiO₂-Teilchen andere anorganische teilchenförmige Stoffe mit sphärischer Struktur vorliegen, wobei die sphärischen SiO₂₋Teilchen mit den anderen anorganischen Stoffen definierte Agglomerate mit einer Teilchengröße im Bereich von 0,06 µm bis 5 µm bilden (gemäß WO99/06012).

Ein weiterer besonders wirksamer Zusatz für das erfindungsgemäße Kosmetikum ist eine Wirkstoffzubereitung mit hohem Radikalschutzfaktor mit einem Gehalt an einem durch Extraktion der Rinde von Quebracho blanco und nachfolgender enzymatischer Hydrolyse gewonnenem Produkt, das wenigstens 90 Gew- % Proanthocyanidin-Oligomere und höchstens 10 Gew-% Gallussäure enthält, in Mikrokapseln, sowie einem durch Extraktion gewonnenen Seidenraupenextrakt, der das Peptid Cecropine, Aminosäuren und ein Vitamingemisch enthält, und einem nichtionischen, kationischen oder anionischen Hydro-Gel oder Gemisch von Hydro-Gelen, und einem oder mehreren Phospholipiden, und Wasser.

Dieses Produkt, z.B. der Wirkstoffkomplex gemäß Beispiel 1 oder 2 der WO99/66881, oder in einer Form, die zusätzlich Superoxiddismutase und Cyclodextrine enthält, z.B. gemäß Beispiel 1 der WO 01/26617, die als RPF-Komplex bezeichnet werden, kann die Gesamtwirkung des erfindungsgemäßen Mittels noch weiter verbessern.

Das erfindungsgemäße Kosmetikum enthält weiterhin kosmetische Hilfs- und Trägerstoffe, wie sie üblicherweise in solchen Zubereitungen verwendet werden, z.B. Wasser, Konservierungsmittel, Farbstoffe, Verdickungsmittel, Duftstoffe, Alkohole, Polyole, Ester, Elektrolyte, Gelbildner, polare und unpolare Öle, Polymere, Copolymere, Emulgatoren, Stabilisatoren. Auch geringe Mengen an Pigmenten, wie Eisenoxide oder Titandioxid, können enthalten sein.

Die für die Erfindung eingesetzten Öle können übliche kosmetische Öle sein, wie ein Mineralöl; hydriertes Polyisobuten; synthetisches oder aus Naturprodukten hergestelltes Squalan; kosmetische Ester oder Ether, die verzweigt oder unverzweigt, gesättigt oder ungesättigt sein können; pflanzliche Öle; Siliconöle oder Gemische zweier oder mehrerer davon.

Besonders geeignete Öle sind beispielsweise Mineralöle, Hydrogenated Polyisobuten, Polyisopren, Squalane, Tridecyltrimellitat, Trimethylpropan-triisostearat, Isodecylcitrat, Neopentylglycoldiheptanoat, PPG-15-stearylether, Cyclomethicone sowie pflanzliche Öle, wie Calendulaöl, Jojobaöl, Avocadoöl, Macadamianußöl, Rizinusöl, Weizenkeimöl, Traubenkernöl, Kukuinußöl, Distelöl, Nachtkerzenöl, Safloröl oder ein Gemisch mehrerer davon. Je nachdem welche Öle ausgewählt werden, werden die kosmetischen Eigenschaften beeinflußt, wie Transparenzgrad, Weichheit, Spreitungswirkung.

Als Ester oder Ether sind zum Beispiel geeignet Dipentaerythrityl hexacaprilate/hexacaprate/tridecyl trimellitate/tridecyl stearate/neopentyl glycol dicaprylate dicaprate, Propylene glycol dioctanoate 5, Propylene glycol dicaprylate 2,30 dicaprate, Tridecyl stearate/neopentyl glycol dicaprylate dicaprate/tridecyl trimellitate, Neopentyl glycol dioctanoate, Isopropyle myristate, Diisopropyl dimer dilinoleate, Trimethylpropane triisostearate, Myristyl ether, Stearyl ether, Cetearyl octanoate, Butyl ether, Dicaprylyl ether, PPG1-PEG9 Lauroyl glycol ether, PPG15 Stearyl ether, PPG14 Butyl ether, Fomblin HC25.

Einwertige höhere Alkohole wie Cetearylalkohol oder mehrwertige Alkohole (Polyole) sind ebenfalls mögliche Bestandteile des erfindungsgemäßen Kosmetikums. Dies sind z.B Propylenglycol, Dipropylenglycol, Ethylenglycol, Isoprenglycol, Glycerin, Sorbitol und Gemische davon. Der Anteil des Polyols liegt im Bereich von 0,1 to 40 Gew-%, vorzugsweise von etwa 5% bis etwa 20 Gew-% der Zusammensetzung.

Kosmetische Zusammensetzungen mit der erfindungsgemäßen Wirkstoffzubereitung können als O/W- oder W/O-Emulsionen vorliegen. Geeignete Emulgatoren für O/W- oder W/O-Emulsionen sind bekannt und sind beispielsweise Anlagerungsprodukte von Ethylenoxid an Fettsäuren, Ester oder Öle.

Die Zusammensetzung kann auch als Gel vorliegen. Zu kosmetischen geeigneten Gelbildnern gehören Carbomer, Xanthangummi, Carrageenan, Akaziengummi, Guargummi, Agar-Agar, Alginate und Tylosen, Carboxymethylcellulose, Hydroxyethylcellulose, quaternisierte Cellulose, quaternisierter Guar, bestimmte Polyacrylate wie Acrylates/C10-30 Alkyl Acrylate Crosspolymer, Polyvinylalkohol, Polyvinylpyrrolidon.

Die Verwendung des erfindungsgemäßen kosmetischen Präparates kann z.B. erfolgen in Gelen, Tagescremes, Nachtcremes, Masken, Körperlotionen, Reinigungsmilch, Grundierungen zur Aufhellung, Make-up's, Deo-Produkten, Körperpuder (mit teilchenförmigem Extrakt), als Anti-Spot-Mittel. Die Herstellung derartiger Produkte erfolgt auf eine Weise, wie sie dem Fachmann auf diesem Gebiet bekannt ist.

Die Erfindung soll nachstehend durch Beispiele näher erläutert werden. Alle Angaben erfolgen in Gewichtsprozent, sofern nichts anderes angegeben ist.

### Beispiel 1 Aufhellungscreme I

| **Phase A** | |
|---|---|
| Wasser | q.s. ad 100 |
| Glycerin | 3,0 |
| EDTA Dinatriumsalz | 0,5 |

| **Phase B** | |
|---|---|
| Tribehenin PEG 20 Ester | 1,0 |
| Stearic acid PEG 100 - Stearate/Glycerylstearate 2,0 | 1,5 |
| Babassu oil | 1,5 |

| **Phase C** | |
|---|---|
| Cyclohexasiloxane | 3,0 |

| **Phase D** | |
|---|---|
| RPF Komplex* | 0,5 |
| Pflanzenextraktkomplex (57 % Lotus, 19,992 % Kiwi, 23,0 % Orchidee, 0,008 % Süßholz) | 3,8 |
| Parfümöl | 0,5 |
| Konservierungsmittel | 1,0 |

| | |
|---|---|
| * gemäß WO99/66881 Beispiel 1 | |

Die separat unter Rühren hergestellten Phasen A und B werden auf etwa 75°C erhitzt und unter Rühren zusammengeführt. Danach wird 20 Minuten bei 3000 U/min homogenisiert. Nach dem Abkühlen unter Rühren auf 40°C wird die Phase C zugegeben und dann bei 35°C die Phase D.

### Beispiel 2: Aufhellungscreme II mit SPF 8

| **Phase A** | |
|---|---|
| Wasser | q.s. ad 100 |
| Glycerin | 3,0 |
| Caffein | 0,5 |
| Bambusa Arundinacea (Stengelextrakt) | 1,0 |

| **Phase B** | |
|---|---|
| Stearic acid | 2,0 |
| PEG - 100 Stearate Glyceryl Stearate | 3,0 |
| Butyl-Methoxydibenzolmethane | 3,0 |
| Ethylhexylsalicylate | 2,5 |
| Dicaprylyl Carbonate | 7,0 |

| **Phase C** | |
|---|---|
| RPF-Komplex* | 1,0 |
| Pflanzenextraktkomplex (52 % Lotus, 28,98 % Kiwi 19 % Orchidee, 0,02 % Süßholz) | 1,0 |
| Konservierungsmittel | 1,0 |
| Parfümöl | 0,8 |

| | |
|---|---|
| * gemäß WO99/66881 Beispiel 1 | |

Es wurde wie im Beispiel 1 verfahren.

### Beispiel 3: Aufhellungscreme III mit SPF 8

| **Phase A** | |
|---|---|
| Wasser | q.s. ad 100 |
| Glycerin | 3,0 |
| Caffein | 0,5 |
| Bambusa Arundinacea (Stengelextrakt) | 1,0 |

| **Phase B** | |
|---|---|
| Stearic acid | 2,0 |
| PEG - 100 Stearate Glyceryl Stearate | 3,0 |
| Butyl-Methoxydibenzolmethane | 3,0 |
| Ethylhexylsalicylate | 2,5 |
| Dicaprylyl Carbonate | 7,0 |

| **Phase C** | |
|---|---|
| Pflanzenextraktkomplex (52 % Lotus, 28,98 % Kiwi 19 % Orchidee, 0,02 % Süßholz) | 1,0 |
| Konservierungsmittel | 1,0 |
| Parfümöl | 0,8 |

Es wurde wie im Beispiel 1 verfahren.

### Beispiel 4: Körpercreme

| **Phase A** | |
|---|---|
| Wasser | q.s. ad 100 |
| Propylenglycol | 3,0 |
| PVM/MA Decadiene Crosspolymer | 0,8 |
| Acrylates/C10-30 Alkyl-acrylate Crosspolymer | 0,25 |

| **Phase B** | |
|---|---|
| Triethanolamin | 0,9 |

| **Phase C** | |
|---|---|
| Cyclopentasiloxane | 3,5 |

| **Phase D** | |
|---|---|
| RPF-Komplex mit Cyclodextrine/Retinol** | 0,03 |
| Vitamin-A-Palmitat | 0,2 |
| Pflanzenextraktkomplex (45 % Lotus, 33 % Kiwi 21 % Orchidee, 1 % Süßholz) | 0,6 |
| AOCS*** | 1,5 |
| AMCS⁺ | 0,1 |
| Jadepulver | 0,5 |

| | |
|---|---|
| ** gemäß WO 01/26617 Beispiel 1 *** asymmetrische lamellare Aggregate gemäß WO 94/00109 ⁺ hartmagnetische Bariumhexaferrit-Einkristalle gemäß WO95/03061 Beispiel 2. | |

Alle Rohstoffe werden bei Raumtemperatur hintereinander zugegeben, wobei Phase B als Neutralisationsmittel eingesetzt ist. Anschließend wird das Gemisch gut homogenisiert.

### Beispiel 5 Anwender-Test

20 weibliche Probanden mit dunklem Hauttyp wurden einem mehrwöchigen Test unter Anwendung einer Aufhellungscreme gemäß Beispiel 1 unterzogen. Zwei separate Hautareale von 2x3 cm wurden auf dem linken Unterarm abgegrenzt. Auf einem Hautareal wurde die Aufhellungscreme gemäß Beispiel 1 (Creme K) aufgetragen, und auf dem anderen Hautareal eine Creme, die die gleichen Komponenten wie im Beispiel 1 enthielt mit Ausnahme von Lotusextrakt, Kiwi-Extrakt und Orchideenextrakt (Creme S). Beide Cremes wurden auf den jeweiligen Hautarealen einmal täglich aufgetragen. Kontrolluntersuchungen erfolgten zum Zeitpunkt 0, +2 Wochen, +4 Wochen und +8 Wochen. Die Probanden vermieden UV-Einstrahlung auf dem Unterarm.

Bei den Kontrolluntersuchungen wurde von dem Tester eine Farbpalette mit 8 verschiedenen Brauntönen von sehr hell (100% Aufhellung) nach sehr dunkel (0% Aufhellung) gegen die behandelten Hautareale gehalten und optisch verglichen.
Die Stufen von 0 nach 100% waren festgelegt mit
8 = 0% Aufhellung
7 = 15%
6 = 30%
5 = 50 %
4 = 65%
3 = 80%
2 = 90%
1 = 100%

### Creme S:

Von den 20 Probanden zeigten 60 % eine Aufhellung um 65 % (Braun ton 4); 25% eine Aufhellung um 50 % (Braunton 5) und 15 % eine Aufhellung um 80% (Braunton 3) nach +8 Wochen. Nach Beendigung der Behandlung und weiteren 8 Wochen zum Zeitpunkt +16 Wochen hatten 90 % der Probanden wieder den Ausgangswert (Braunton 8) und 10 % den Wert 15% (Braunton 7) erreicht.

### Creme K:

Von den 20 Probanden zeigten 50 % eine Aufhellung um 65 %; 35% eine Aufhellung um 80 % und 15 % eine Aufhellung um 90% nach +8 Wochen. Nach Beendigung der Behandlung und weiteren 8 Wochen zum Zeitpunkt +16 Wochen hatte keiner der Probanden wieder den Ausgangswert erreicht, 45 % hatten den Wert 50% (Braunton 5) und 55% den Wert 30% (Braunton 6) erreicht.

Das Ergebnis zeigt deutlich, dass die erfindungsgemäße Creme K eine bessere Aufhellungswirkung insgesamt zeigte. Besonders signifikant ist jedoch die deutlich geringere Abschwächung der Hautaufhellung nach Beendigung der Behandlung, so dass von einer besonders langanhaltenden Wirkung gesprochen werden kann.

## Patentansprüche

1. Kosmetisches Aufhellungs- und Reinigungsmittel für die Haut, **dadurch gekennzeichnet, dass** das Mittel einen Komplex aus Pflanzenextrakten, bestehend aus
38-62 Gew-% eines Extraktes aus Lotusblüten,
22-42 Gew-% Kiwi-Fruchtextrakt,
15-26 Gew-% Extrakt der weißen Orchidee Phalaenopsis amabilis und
0,001-2 Gew-% Süßholzextrakt
umfasst, bezogen auf das Trockengewicht des Komplexes, der Komplex einem Anteil von 0,5 bis 5 Gew-% des Mittels einnimmt, und der Rest bis zu 100 Gew-% übliche kosmetische Trägerstoffe, Hilfsstoffe, andere Wirkstoffe oder Gemische davon sind.

2. Mittel nach Anspruch 1, **dadurch gekennzeichnet, dass** der Komplex 46-55 Gew-% Lotusextrakt enthält.

3. Mittel nach Anspruch 1, **dadurch gekennzeichnet, dass** der Komplex 27-37 Gew-% Kiwi-Fruchtextrakt enthält.

4. Mittel nach Anspruch 1, **dadurch gekennzeichnet, dass** der Komplex 17-21 Gew-% Orchideenextrakt enthält.

5. Mittel nach Anspruch 1, **dadurch gekennzeichnet, dass** der Komplex 0,005-0,9 Gew-% Süßholzextrakt enthält, vorzugsweise 0,01-0,5 Gew-%.

6. Mittel nach Anspruch 1, **dadurch gekennzeichnet, dass** es als weiteren Wirkstoff einen Radikalfänger enthält.

7. Mittel nach Anspruch 1, **dadurch gekennzeichnet, dass** es als weiteren Wirkstoff einen UVA-Sonnenschutzfilter, einen UVB-Sonnenschutzfilter oder ein Gemisch davon enthält.

8. Mittel nach Anspruch 7, **dadurch gekennzeichnet, dass** das Gemisch von UVA-Sonnenschutzfilter und UVB-Sonnenschutzfilter im Verhältnis 1:0,6-1,4 vorliegt.

9. Mittel nach Anspruch 1, **dadurch gekennzeichnet, dass** es als weiteren Wirkstoff eine Kombination von hartmagnetische Teilchen aus Bariumhexaferrit-Einkristallen mit einer Teilchengröße im Bereich von 100 bis 350 nm und einer Koerzitivkraft der Teilchen im Bereich von 300.000 bis 1.200.000 A/m und gemahlenem Jadestein mit einer Teilchengröße im Bereich von 50 - 95 nm enthält.

## Claims

1. Cosmetic whitening and cleansing agent for the skin,
comprising a complex of plant extracts, consisting of
38-62 wt.% of Lotus flower extract,
22-42 wt.% of Kiwi fruit extract,
15-26 wt.% of an extract of the White Orchid Phalaenopsis amabilis, and
0.001-2 wt.% of an extract of liquorice,
related to the dry weight of the complex, the complex making up a percentage of 0.5-5 wt.% of the agent, and the remainder of the ingredients up to 100 wt.% of the complex being usual cosmetic carriers, auxiliary agents, other effective cosmetic agents or mixtures thereof.

2. Agent according to claim 1, wherein the complex contains 46-55 wt.% of Lotus extract.

3. Agent according to claim 1, wherein the complex contains 27-37 wt.% of Kiwi fruit extract.

4. Agent according to claim 1, wherein the complex contains 17-21 wt.% of Orchid extract.

5. Agent according to claim 1, wherein the complex contains 0.005-0.9 wt.% of liquorice extract, preferably 0.01-0.5 wt.%.

6. Agent according to claim 1, wherein the complex contains a radical scavenger as a further effective agent.

7. Agent according to claim 1, wherein the complex contains a UVA sun screen filter, a UVB sun screen filter or a mixture of both, as a further effective agent.

8. Agent according to claim 7, wherein the mixture of UVA and UVB sun screen filters is present in a ratio of 1 : 0.6-1.4

9. Agent according to claim 1, wherein the complex contains a combination of hard magnetic particles of barium hexaferrit single crystals having a particle size ranging from 100 to 350 nm and a particles' coercive force ranging from 300,000 to 1,200,000 A/m, and ground jade stone having a particle size ranging from 50 to 95 nm, as a further effective agent.

## Revendications

1. Agent cosmétique de blanchiment et de nettoyage de la peau, **caractérisé en ce que** l'agent comprend un complexe d'extrait de plantes constitué de
38 à 62 % en poids d'un extrait de fleurs de lotus,
22 à 42 % en poids d'extrait de kiwi,
15 à 26 % en poids d'extrait d'orchidée blanche *Phalaenopsis amabilis* et
0,001 à 2 % en poids d'extrait de réglisse, par rapport au poids sec du complexe, le complexe acceptant une proportion de 0,5 à 5 % en poids de l'agent, et le reste allant jusqu'à 100 % en poids de véhicules cosmétiques, adjuvants classiques, autres principes actifs ou leurs mélanges.

2. Agent selon la revendication 1, **caractérisé en ce que** le complexe contient 46 à 55 % en poids d'extrait de lotus.

3. Agent selon la revendication 1, **caractérisé en ce que** le complexe contient 27 à 37 % en poids d'extrait de kiwi.

4. Agent selon la revendication 1, **caractérisé en ce que** le complexe contient 17 à 21 % en poids d'extrait d'orchidée.

5. Agent selon la revendication 1, **caractérisé en ce que** le complexe contient 0,005-0,9 % en poids d'extrait de réglisse, de préférence, 0,01 à 0,5 % en poids.

6. Agent selon la revendication 1, **caractérisé en ce qu'**il contient comme autre principe actif, un piégeur de radicaux.

7. Agent selon la revendication 1, **caractérisé en ce qu'**il contient comme autre principe actif, un filtre de protection solaire contre les UVA, un filtre de protection solaire contre les UVB ou un mélange de ceux-ci.

8. Agent selon la revendication 7, **caractérisé en ce que** le mélange de filtre de protection solaire contre les UVA et le filtre de protection solaire contre les UVB se présente en un rapport de 1:0,6-1,4.

9. Agent selon la revendication 1, **caractérisé en ce qu'**il contient comme autre principe actif, une combinaison de particules magnétiques dures de monocristaux d'hexaferrite de baryum présentant une taille de particules de l'ordre de 100 à 350 nm et une force coercitive des particules de l'ordre de 300 000 à 1 200 000 A/m et du jade broyé présentant une taille de particules de l'ordre de 50 à 95 nm.
